Europäisches Patentamt

European Patent Office

Office européen des brevets

(11) Veröffentlichungsnummer: **0 009 672**
**B1**

(12) **EUROPÄISCHE PATENTSCHRIFT**

(45) Veröffentlichungstag der Patentschrift:
**09.12.81**

(21) Anmeldenummer: **79103392.1**

(22) Anmeldetag: **11.09.79**

(51) Int. Cl.³: **A 61 B 5/14**

(54) **Vorrichtung für die kutane Messung des Partialdruckes von Gasen in Blut.**

(30) Priorität: **11.09.78 CH 9488/78**

(43) Veröffentlichungstag der Anmeldung:
**16.04.80 Patentblatt 80/8**

(45) Bekanntmachung des Hinweises auf die Patenterteilung:
**09.12.81 Patentblatt 81/49**

(84) Benannte Vertragsstaaten:
**CH DE FR GB IT NL SE**

(56) Entgegenhaltungen:
**DE-A-2 255 879**
**DE-A-2 305 049**
**GB-A-2 014 738**
**GB-A-2 014 739**
**US-A-3 985 633**

(73) Patentinhaber: **F. HOFFMANN-LA ROCHE & CO.**
**Aktiengesellschaft, CH-4002 Basel (CH)**

(72) Erfinder: **Eberhard, Patrick, Dr., Parkallee 11,**
**CH-4123 Allschwil (CH)**
Erfinder: **Mindt, Wolfgang, Dr., Sonnmattstrasse 25,**
**CH-4142 Münchenstein (CH)**

(74) Vertreter: **Wuesthoff, Franz, Dr.-Ing. et al, Patentanwälte**
**Wuesthoff -v. Pechmann-Behrens-Goetz**
**Schweigerstrasse 2, D-8000 München 90 (DE)**

Vorrichtung für die kutane Messung des Partialdruckes von Gasen in Blut

Die Erfindung betrifft eine Vorrichtung zur kutanen Bestimmung der Perfusion und des Partialdruckes von Gasen in Blut unter Verwendung einer Heizeinrichtung und polarographischen Blutgaselektroden, mit einer Anode und mit der Anode eine bauliche Einheit bildenden Kathoden, wobei eine Kathode zur Bestimmung des Partialdruckes eines Blutgases dient (DE-A-22 55 879). Mit «kutan» soll hier eine unblutige Messwertaufnahme durch eine auf die Haut aufgesetzte polarographische Elektrode bezeichnet werden.

Es ist bekannt, dass der mit geheizten kutanen Sauerstoffelektroden gemessene $pO_2$-Wert nicht immer mit dem arteriellen $pO_2$-Wert korreliert, da auch andere Parameter wie z.B. die Mikrozirkulation, Diffusionswiderstand der Haut und der Kreislaufzustand den kutanen $pO_2$-Wert beeinflussen können. So verursachen beispielsweise einige in der Intensivpflege verwendete Medikamente, die gefässerweiternde oder -verengende Wirkung haben, Schwankungen des kutanen $pO_2$-Werts, währenddessen der zentral-arterielle $pO_2$-Wert konstant bleibt. Ebenfalls ist bekannt, dass Schwankungen des Blutdrucks (insbesondere bei Patienten mit labilem Kreislaufzustand) den kutanen $pO_2$-Wert beeinflussen können. Aus diesen Gründen ist eine einwandfreie Interpretation des kutanen $pO_2$-Werts nicht immer möglich. Diese Beschränkung hat wesentlich dazu beigetragen, dass sich die Methode der kutanen $pO_2$-Messung bei der Überwachung von erwachsenen Intensivpatienten bisher nicht durchgesetzt hat.

Es wurden bereits Versuche unternommen, mit der kutanen $pO_2$-Messung eine Perfusionsmessung zu koppeln, um eine verbesserte Interpretation des kutanen $pO_2$-Werts zu ermöglichen. Dies erfolgte bisher ausschliesslich durch Methoden, die auf der Ermittlung des Wärmetransports an der Messstelle beruhen. Die von Lübbers und Mitarbeitern beschriebene Methode (Deutsche Auslegeschrift 2 255 879) beruht auf der Messung der Heizleistung die zur Erwärmung der Sauerstoffelektrode auf eine konstante Temperatur benötigt wird. Mit zunehmender lokaler Druchblutung wird eine grössere Heizleistung verbraucht. Ein Nachteil dieser Methode ist jedoch, dass nur ein kleiner Teil der Heizleistung (nach kürzlich publizierten Schätzungen 25%) durch den Blutfuss abgeführt wird. Der Rest der Heizleistung wird zur Erwärmung von nicht durchblutetem Gewebe sowie der Umgebung der Elektrode verbraucht. Aus diesem Grund ist die Methode nach Lübbers et al. relativ unempfindlich und erfordert ausserdem einen hohen Aufwand zur Wärmeisolierung der Elektrode gegen die Umgebung. Es ist jedoch anzunehmen, dass prinzipiell durch ein simultane Messung der Perfusion die Wertigkeit der kutanen $pO_2$-Überwachung erhöht wird.

Ausser der von Lübbers beschriebenen Methode durch Bestimmung der Heizleistung zur Aufrechterhaltung einer konstanten Temperatur an der Messstelle gibt es noch andere Methoden, die auf der Messung des Wärmetransports beruhen. Eine andere Methode besteht beispielsweise darin, an einer Stelle Wärme zuzuführen und an einer zweiten, in bestimmtem Abstand davon gelegenen Stelle die Temperatur zu messen. Die sich ergebene Temperaturdifferenz ist ebenfalls ein Mass für die Perfusion.

Die vorstehenden Ausführungen zum Stand der Technik beziehen sich praktisch ausschliesslich auf die Messung des $pO_2$ im Blut. Dies ist darauf zurückzuführen, dass bis heute nur die $pO_2$-Messung Eingang in die klinische Praxis gefunden hat. Es gibt jedoch Vorschläge (vgl. z.B. DE-A-23 05 049), wonach der Partialdruck anderer Blutgase, insbesondere der $pCO_2$, in prinzipiell gleicher Weise gemessen werden kann. Dabei sind solche Messungen entweder getrennt oder miteinander kombiniert durchführbar. Im Hinblick darauf ist die vorliegende Erfindung auch nicht auf die Messung einer bestimmten Blutgaskonzentration beschränkt, sondern sie ist ausser auf die Messung von $pO_2$ auch auf die von anderen elektrochemisch zu bestimmenden Gasen im Blut, insbesondere die von $pCO_2$ anwendbar.

Der Erfindung liegt die Aufgabe zugrunde, eine verbesserte Methode zur simultanen, kutanen Messung des Partialdrucks eines Gases im Blut und der Perfusion aufzufinden, die nicht die Nachteile der bekannten Methoden aufweist, d.h., mit der insbesondere höhere Genauigkeit erzielbar ist.

Erfindungsgemäss wird dies dadurch erreicht, dass die Vorrichtung zwei Kathoden aufweist, von denen die zweite Kathode im Verhältnis zur ersten, zur Bestimmung des Partialdruckes des betreffenden Blutgases dienenden, einen sehr niedrigen Blutgaseigenverbrauch aufweisenden Kathode derart ausgebildet ist, dass sie einen wesentlich grösseren Blutgaseigenverbrauch aufweist und zur Bestimmung der Blutgasverfügbarkeit an der Auflagefläche der Vorrichtung dient.

Vorteilhafte Ausgestaltungen ergeben sich aus den Unteransprüchen und der Erläuterung eines Ausführungsbeispiels. Wie bereits erwähnt, kann mit einer solchen Elektrodenvorrichtung die Korrelation des kutan gemessenen Blutgaswertes mit dem tatsächlichen arteriellen Wert überwacht werden, indem die Messwerte für die Gasverfügbarkeit an der Auflagefläche zur Interpretation des gemessenen Konzentrationswertes dienen können. Mit anderen Worten kann aus dem Messwert der Gasverfügbarkeit ein Korrekturfaktor für den Messwert des kutanen $pO_2$ ermittelt werden. Daneben kann der gleichzeitig gemessene Wert der Gasverfügbarkeit als Funktionskontrolle für die Vorrichtung dienen. So lassen sich beispielsweise Fehler in der Applikation, etwa die Verwendung von zuviel Kontaktgel, die Ablösung des Sensors, das Vorhandensein von Luftblasen zwischen Haut und Sensor, Brandblasen und andere Hautschäden, etc. erkennen. Andererseits lassen sich bei Sicherstellung richtiger Funktion Änderungen der Kreislauftätigkeit erfassen. Insbesondere sind die Auswirkungen von Medikamenten auf den Kreislauf erfassbar, so dass unter anderem ihr Einfluss auf die Messung der Blutgaskonzentration berücksichtigt werden kann. Schliesslich ist vor allem bei Neugeborenen, bei denen üblicherweise kein medikamentöser Einfluss vorhanden ist, mit Hilfe der Messung der Gasverfügbarkeit, die auch vom Blutdruck abhängig ist, eine qualitative Überwachung des Blutdruckes möglich.

Im folgenden wird anhand der beiliegenden Zeichnung ein Ausführungsbeispiel der Erfindung beschrieben.

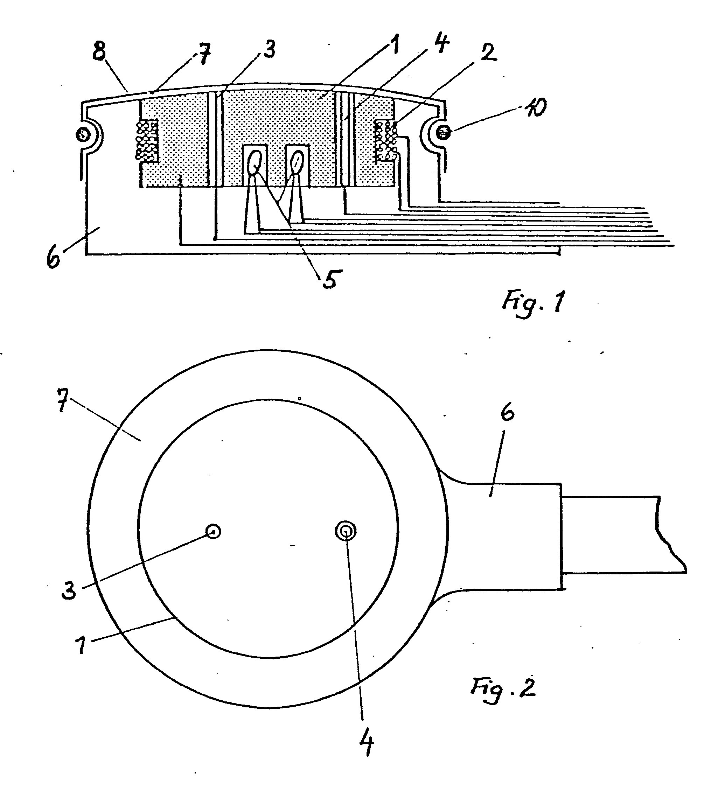

Die Zeichnung zeigt in Fig. 1 einen Schnitt durch eine Sauerstoffelektrode nach der Erfindung und in Fig. 2 eine Ansicht der Auflagefläche ohne Membran.

Bei dieser Ausführungsform ist eine im wesentlichen zylindrische Silberelektrode 1 mit einer Heizungseinrichtung versehen. Die Mantelfläche der Silberelektrode 1 weist eine umlaufende Vertiefung auf, in der sich eine Heizwicklung 2 befindet. Zwei exzentrische achsparallel angeordnete durchgehende Bohrungen in der Silberelektrode dienen zur Aufnahme zweier Platinelektroden 3 und 4. Die Enden der Platinelektroden liegen in einer Ebene mit der entsprechenden Stirnfläche der Silberelektrode. Die eine der beiden Platinelektroden 3 besteht aus sehr dünnem Platindraht und hat einen Durchmesser von etwa 25 μm. Die andere Platinelektrode 4 besteht aus einem Platinstift von ca. 2 mm Durchmesser. Von der dieser Stirnfläche gegenüberliegenden Seite der Silberelektrode sind zwei weitere Bohrungen in den Silberblock geführt, in der sich je ein Temperaturmesselement 5 befindet.

Anstelle von Platin als Elektrodenmaterial könnte auch ein anderes bei polarographischen Elektroden übliches Edelmetall, beispielsweise Gold, Verwendung finden.

Alle Zuleitungen sind seitlich weggeführt und zu einem Kabel (nicht gezeigt) zusammengefasst. Die gesamte Anordnung ist mit einer flachzylindrischen Kapsel 6 aus Kunststoff umgeben, wobei eine Stirnfläche der Kapsel die Elektroden frei lässt. Zur Messung ist die Stirnfläche mit einem Elektrolytfilm 7 überzogen und mit einer Membran 8 abgedeckt, die durch einen elastischen O-Ring 10 gehalten ist. Die Membran besteht aus Polytetrafluoräthylen (Teflon) (Warenzeichen) von 25 μm Dicke. Es sind auch andere Materialien und Dicken für die Membran denkbar. Wichtig ist lediglich, dass die Membran für Sauerstoff relativ gut durchlässig sein muss.

Im Betrieb sind die Elektroden so geschaltet, dass die beiden Platinelektroden 3 und 4 als Kathoden, die Silberelektrode 1 als Anode dient. Die Mikrokathode 3, die einen Durchmesser von ca. 25 μm hat, weist einen sehr niedrigen Sauerstoffeigenverbrauch auf. Sie eignet sich infolgedessen zur Messung des Sauerstoffpartialdrucks. Die grossflächige Kathode 4, die einen Durchmesser von 2 mm besitzt, verbraucht demzufolge praktisch den gesamten bei der Auflagefläche ankommenden Sauerstoff. Infolgedessen kann sie zur Bestimmung der Sauerstoffverfügbarkeit dienen. Auf diese Weise ist also simultan mit der kutanen $pO_2$-Messung eine Aussage über die Blutperfusion in der Umgebung der Messstelle zu erhalten. Der Vorteil der Sauerstoff-Flussmessung gegenüber der bisher in diesem Zusammenhang verwendeten Wärmeflussmessung besteht darin, dass die Perfusionsmessung nicht durch Abführung von Wärme in die Umgebung gestört wird. Es ist demzufolge keine aufwendige thermische Isolation der Messanordnung nötig. Ausserdem besitzt die Messung des Sauerstoffflusses eine höhere Empfindlichkeit.

Neben der vorstehend beschriebenen bevorzugten Ausführungsform der Erfindung sind auch andere Ausführungsformen mit konstruktiv verschiedenen Lösungen denkbar. So kann beispielsweise die Heizung anstatt an der Anode an der grossflächigen Kathode 4 angeordnet sein, die zu diesem Zweck entsprechend vergrössert werden muss.

Eine andere Möglichkeit besteht darin, ein von den Elektroden völlig unabhängiges Heizelement vorzusehen.

Eine weitere Möglichkeit, den unterschiedlich hohen Sauerstoffverbrauch der beiden Kathoden zu erreichen, besteht darin, zwei gleich grosse sogenannte Makrokathoden vorzusehen und diese mit unterschiedlich durchlässigen Membranen abzudecken. Die eine der beiden, die zur Perfusionsmessung dient, kann eine Teflonmembran besitzen, deren Druchlässigkeit hoch ist, während die andere, zur $pO_2$-Messung dienende Kathode mit einer relativ wenig durchlässigen Membran, z.B. Mylar, abgedeckt sein muss. Diese Anordnung wäre vom Aufbau und vor allem von der Handhabung her komplizierter als die vorstehend beschriebene bevorzugte Ausführungsform.

**Patentansprüche**

1. Vorrichtung zur kutanen Bestimmung der Perfusion und des Partialdruckes von Gasen in Blut unter Verwendung einer Heizeinrichtung (2) und polarographischen Blutgaselektroden (1, 3, 4), mit einer Anode (1) und mit der Anode (1) eine bauliche Einheit bildenden Kathoden (3, 4), wobei eine Kathode (3) zur Bestimmung des Partialdruckes eines Blutgases dient, dadurch gekennzeichnet, dass die Vorrichtung zwei Kathoden (3, 4) aufweist, von denen die zweite Kathode (4) im Verhältnis zur ersten, zur Bestimmung des Partialdruckes des betreffenden Blutgases dienenden, einen sehr niedrigen Blutgaseigenverbrauch aufweisenden Kathode (3) derart ausgebildet ist, dass sie einen wesentlich grösseren Blutgaseigenverbrauch aufweist und zur Bestimmung der Blutgasverfügbarkeit an der Auflagefläche der Vorrichtung dient.

2. Vorrichtung nach Anspruch 1, dadurch gekennzeichnet, dass die erste Kathode (3) eine sogenannte Mikrokathode mit einem Durchmesser von ca. 25 µm und die zweite Kathode (4) eine grossflächige Kathode mit einem Durchmesser von ca. 2 mm ist.

3. Vorrichtung nach Anspruch 1, dadurch gekennzeichnet, dass die beiden Kathoden mit unterschiedlichen Membranen abgedeckte grossflächige Kathoden sind und die Gasdurchlässigkeit der Membran über der ersten Kathode kleiner als die der Membran über der zweiten Kathode ist.

## Claims

1. Apparatus for the cutaneous determination of the perfusion and of the partial pressure of gases in blood using a heating arrangement (2) and polarographic blood gas electrodes (1, 3, 4), with one anode (1) and cathodes (3, 4) forming a structural unit with the anode (1), whereby one cathode (3) serves for the determination of the partial pressure of a blood gas, characterized in that the apparatus has two cathodes (3, 4), of which the second cathode (4) in relation to the first cathode (3), which serves for the determination of the partial pressure of the respective blood gas and which has a very low blood gas consumption, is formed so that it has a substantially larger blood gas consumption and serves for the determination of the blood gas availability at the contact surface of the apparatus.

2. Apparatus according to claim 1, characterized in that the first cathode (3) is a so-called microcathode with a diameter of about 25 µm and the second cathode (4) is a large surface cathode with a diameter of about 2 mm.

3. Apparatus according to claim 1, characterized in that the two cathodes are large surface cathodes covered with different membranes and the gas permeability of the membrane over the first cathode is smaller than that of the membrane of the second cathode.

## Revendications

1. Dispositif pour la détermination cutanée de la perfusion et de la pression partielle de gaz dans le sang par utilisation d'un dispositif de chauffage (2) et d'électrodes polarographiques pour gaz du sang (1, 3, 4), comportant une anode (1) et des cathodes (3, 4) formant avec l'anode (1) une unité de construction, une cathode (3) servant à déterminer la pression partielle d'un gaz du sang, caractérisé en ce que le dispositif comporte deux cathodes (3, 4) dont la deuxième (4), en relation avec la première cathode (3), qui sert à déterminer la pression partielle du gaz sanguin en question et consomme elle-même très peu du gaz sanguin, est conçue de manière à consommer elle-même beaucoup plus de gaz sanguin et sert à déterminer le gaz sanguin disponible à la surface d'application du dispositif.

2. Dispositif selon la revendication 1, caractérisé en ce que le première cathode (3) est une micro-cathode dont le diamètre est d'environ 25 microns et la deuxième cathode (4) est une cathode à grande surface à un diamètre d'environ 2 mm.

3. Dispositif selon la revendication 1, caractérisé en ce que les deux cathodes sont des cathodes de grandes surfaces comportant en écrans des membranes différentes et en ce que la perméabilité aux gaz de la membrane qui recouvre la première cathode est inférieure à celle de la membrane de la deuxième cathode.

Fig. 1

Fig. 2